# EUROPÄISCHE PATENTSCHRIFT

(11) **EP 0 840 722 B1**
(45) Veröffentlichungstag und Bekanntmachung des Hinweises auf die Patenterteilung: **05.04.2000**
(21) Anmeldenummer: 96925694.0
(22) Anmeldetag: 09.07.1996
(51) Int. Cl.: C07C 273/18, C07C 275/62, B01J 10/00

(54) **VERFAHREN UND VORRICHTUNG ZUR HERSTELLUNG VON (CYCLO)ALIPHATISCHE BIURETGRUPPEN ENTHALTENDEN POLYISOCYANATEN**
PROCESS AND DEVICE FOR PREPARING (CYCLO)ALIPHATIC BIURET GROUPS-CONTAINING POLYISOCYANATES
PROCEDE ET DISPOSITIF DE PREPARATION DE POLYISOCYANATES CONTENANT DES GROUPES BIURETS (CYCLO)ALIPHATIQUES

(30) Priorität: 12.07.1995 DE 19525474
(43) Veröffentlichungstag der Anmeldung: 13.05.1998
(73) Patentinhaber: BASF AKTIENGESELLSCHAFT, 67056 Ludwigshafen (DE)
(72) Erfinder: HEIDER, Wolfgang, D-67434 Neustadt (DE); WOLFF, Stefan, D-67117 Limburgerhof (DE); BRUCHMANN, Bernd, D-67069 Ludwigshafen (DE); BITTINS, Klaus, D-67227 Frankenthal (DE)
(74) Vertreter: Isenbruck, Günter, Dr.
(86) Internationale Anmeldenummer: EP9603003
(87) Internationale Veröffentlichungsnummer: WO9703044

(56) Entgegenhaltungen:
- EP-A- 0 251 952
- EP-A- 0 259 233
- DE-A- 1 543 178
- DE-A- 1 931 055
- DE-A- 3 403 277

## Beschreibung

Die Erfindung betrifft ein Verfahren und eine Vorrichtung zur Herstellung von Biuretgruppen enthaltenden Polyisocyanaten aus (cyclo)aliphatischen Diisocyanaten unter Verwendung von Wasserdampf oder einer wasserabspaltenden Substanz als Reaktanden, die in einem Rührreaktor miteinander vermischt werden.

Biuretgruppen aufweisende (cyclo)aliphatische Polyisocyanate werden u.a. in hochwertigen licht- und wetterbeständigen Zweikomponenten-PUR-Lacken eingesetzt. Auch andere Anwendungen, wie Klebstoffe und Dispersionen sind bekannt. Eine Literaturübersicht ist der DE 34 03 277 zu entnehmen. Zur Herstellung von Biuretgruppen aufweisenden (cyclo)aliphatischen Polyisocyanaten werden die Diisocyanate mit einer bestimmten Menge an Biuretisierungsmittel (Wasser oder wasserabspaltende Substanz) bei einer Temperatur zwischen 100 und 200°C umgesetzt. Anschließend wird von dem so entstandenen Rohprodukt das überschüssige monomere Diisocyanat durch einer mehrstufige Destillation abgetrennt.

Bei Verwendung von t-BuOH (oder einer anderen wasserabspaltenden Substanz) als Biuretisierungsmittel wird das entstandene Urethan katalytisch in Isobuten, Kohlendioxid und eine Isocyanatoamin-Zwischenstufe gespalten. Dazu sind jedoch hohe Reaktionstemperaturen (>140°C) notwendig und die Reaktionsprodukte verfärben sich gelb. Dies ist ungünstig, denn aus anwendungstechnischen Gründen werden z.B. beim Klarlack farblose Produkte gefordert. Außerdem entstehen bei der Verwendung von wasserabspaltenden Substanzen (z.B. OH-Gruppen haltige Moleküle) als Biuretisierungsmittel Nebenprodukte, die keine Biuretstruktur haben und die Lagerstabilität des Wertproduktes verschlechtern oder andere verfahrenstechnische Probleme bewirken. Daher wird Wasser als Biuretisierungsmittel bevorzugt. Allerdings entstehen während der Reaktion in der Regel unlösliche Harnstoffe und die erhaltenen Produkte weisen eine schlechte Lagerstabilität bezüglich der Monomerenrückspaltung auf. Dadurch wird der kennzeichnungspflichtige Grenzwert von 0,5% freien monomeren Diisocyanates schnell überschritten, insbesondere bei Lagerung oberhalb der Raumtemperatur. Zur Vermeidung dieser Nachteile sind einige Vorschläge bekannt (siehe beispielsweise EP 259 233 und EP 251 952. Hier wird die Verwendung von katalytischen Mengen von Protonensäuren zur Vermeidung der Nebenproduktbildung bei der Synthese von Biuretgruppen aufweisenden aliphatischen und cycloaliphatischen Polyisocyanaten beschrieben.

Trotzdem gelingt es beim Einsatz von üblichen Rührbehältern nicht, das als Biuretisierungsmittel eingesetzte Wasser vollständig mit dem Diisocyanat zur Reaktion zu bringen. Mit dem bei der Reaktion entstehenden Kohlendioxid entweicht entsprechend dem vorliegenden Partialdruck Wasserdampf und Diisocyanat. Dieses Gasgemisch kondensiert an kalten Stellen im Reaktor und insbesondere im nachgeschalteten Abgaskühler. Dort reagiert das Diisocyanat mit dem Wasserdampf zu Polyharnstoffen, was schließlich zur Verstopfung der Abgasleitungen und des Abgaskühlers führt. Ein zuverlässiger Dauerbetrieb ist auf diese Art nicht möglich.

Der Erfindung liegt daher die Aufgabe zugrunde, eine Reaktionsführung und eine hierfür geeignete Vorrichtung zu schaffen, bei der das entweichende Abgas praktisch frei von Wasserdampf ist und daher keine meßbare Polyharnstoffbildung im Abgassystem auftritt.

Diese Aufgabe wird dadurch gelöst, daß die Reaktanden im Gegenstrom durch einen aus mindestens zwei Stufen bestehenden kaskadenförmigen Rührreaktor geführt werden. Hierdurch wird eine feindisperse Verteilung des eingeleiteten Wasserdampfes bewirkt. Die aufsteigenden Gasblasen verarmen auf ihrem Weg durch die Isocyanatlösung an Wasserdampf und werden an Kohlendioxid angereichert. Durch die Mehrstufigkeit der Kaskade und die in den Rührreaktor eingebauten Störelemente, die ein einfaches Durchströmen des Rührreaktors verhindern, wird die Verweilzeit der Gasblasen in der Flüssigkeit erheblich verlängert, so daß eine vollständige Absorption des Wasserdampfes und damit seine vollständige Umsetzung erreicht werden kann.

Gemäß einer besonders vorteilhaften Ausbildung des erfindungsgemäßen Verfahrens wird die Verteilung des gasförmigen Anteils im flüssigen Reaktanden durch in den Rührreaktor eingebaute Störelemente verbessert. Als Störelemente können mit Abstand voneinander angeordnete, mit zentralen Öffnungen versehene Scheiben verwendet werden. Zusätzlich können in Längsrichtung des Rührreaktors verlaufende Störleisten eingesetzt werden. Dem als Biuretisierungmittel zugeführten Reaktanden, vorzugsweise Wasserdampf, kann 10 bis 95 Vol. % Stickstoff und/oder Kohlendioxid beigemischt werden. Für die Reaktion wird eine Temperatur von 60 bis 200°C, vorzugsweise 100 bis 150°C, eingestellt. Vorzugsweise wird das am Kopfende des Rührreaktors abströmende Abgas mit kaltem (cylco)aliphatischen Diisocyanat gespült, das anschließend dem Verfahren zugeführt wird. Wenn das bei der Reaktion entstehende Abgas über einen Abgaskühler geleitet wird, der zusätzlich mit kaltem Diisocyanat gespült wird, können selbst bei langer Verfahrensdauer keine Polyharnstoffrückstände im Abgassystem nachgewiesen werden.

Zur Durchführung des Verfahrens ist erfindungsgemaß ein Rührreaktor vorgesehen, der einen senkrecht stehenden rohrförmigen Behälter aufweist, in dem parallel zur Längsachse eine Antriebachse drehbar befestigt ist, an der mindestens zwei Scheibenrührer mit Abstand voneinander befestigt sind und bei dem zwischen diesen Scheibenrührern an der Innenwand des Behälters befestigte Stromstörscheiben angeordnet sind, die eine zentrale Öffnung aufweisen. Es hat sich herausgestellt, daß in Abhängigkeit von den Reaktionsparametern zweckmäßigerweise 2 bis 6 Scheibenrührer und 1 bis 5 Stromstörscheiben eingesetzt werden. Das Verhältnis der Öffnung der Stromstörscheiben zu deren Gesamtfläche ergibt sich aus der Rührergröße.

Gemäß einer Weiterbildung des erfindungsgemäßen Rührreaktors können an dessen Innenwand parallel zu seiner Längsachse verlaufende, radial sich nach innen erstreckende Stromstörleisten angeordnet sein. Diese können vorzugsweise mit Abstand zur Innenwand des Reaktors befestigt sein. Vorteilhafterweise werden mindestens 4 im gleichen Winkelabstand voneinander angeordnete Störleisten vorgesehen. Die Stromstörleisten sind genormt, ihre Breite beträgt dabei 0,1 D, wobei D der Durchmesser des Behälters ist.

Der Rührreaktor kann von einem beheizbaren Mantel umschlossen sein. Vorteilhafterweise ist das Kopfende des Rührreaktors mit einem Kühlbehälter verbunden, in dessen unterem Teil ein Kühler eingebaut ist. Oberhalb dieses Kühlers befindet sich ein Injektor für den flüssigen Reaktanden. Diese Einheit mündet in einer Abgaseleitung. Vorteilhafterweise liegt das Verhältnis der Höhe des Rührreaktors zu dessen Durchmesser im Bereich 2 bis 6 und ist vorzugsweise größer als 4,5.

Mit Reaktoren der vorbeschriebenen Bauweise läßt sich das erfingungsgemäße Verfahren besonders vorteilhaft durchführen.

Weitere Einzelheiten und Vorteile der Erfindung können der Beschreibung der in der Zeichnung dargestellten Versuchsanlage entnommen werden. Es zeigen:
- Fig. 1: eine bekannte Anlage mit einem einfachen Rührbehälter,
- Fig. 2: eine erfindungsgemäße Anlage mit kaskadenförmigem Rührreaktor,
- Fig. 3: den erfindungsgemäßen Rührreaktor vergrößert im Schnitt,
- Fig. 4: eine Draufsicht auf einen im Rührreaktor angeordneten Scheibenrührer.

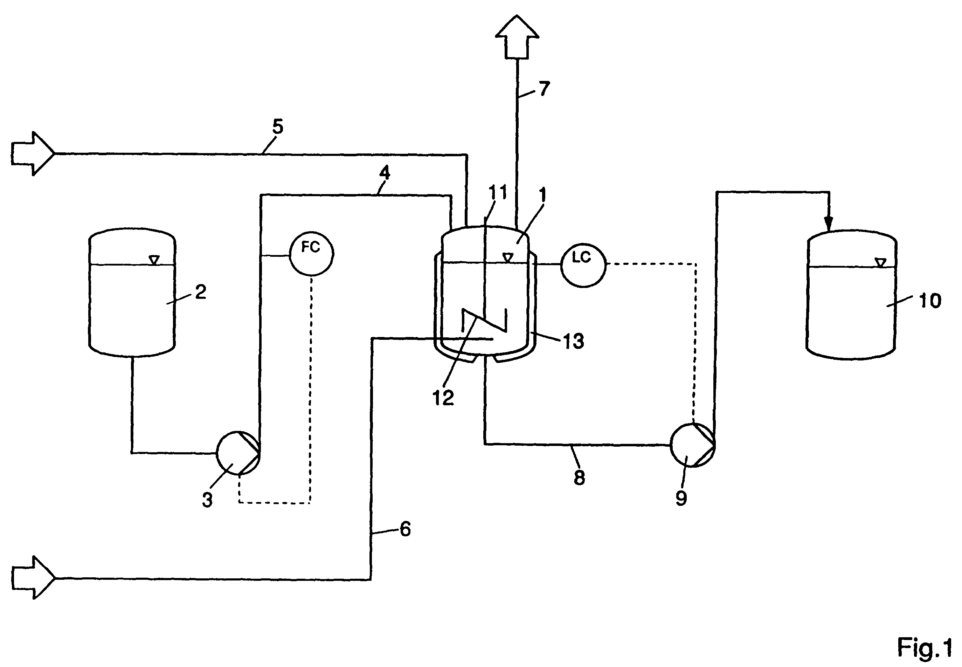

In der in Fig. 1 dargestellten bekannten Anlage erfolgt die Reaktion zur Herstellung von (cyclo)aliphatischen, Biuretgruppen enthaltenden Polyisocyanaten in einem einfachen Rührreaktor 1. Diesem werden aus einem Behälter 2 über eine Pumpe 3 durch eine Leitung 4 Hexamethylendiisocyanat (HDI) als einer der beiden Reaktanden von oben her zugeführt. Durch Leitung 5 können Katalysatoren eingeführt werden, beispielsweise starke anorganische Lewis- oder Brønstedt-Säuren (vgl. DE-A-15 43 178) und/oder Salze aus stickstoffhaltigen Basen und anorganischen und/oder organischen Säuren (vgl. DE-A-19 31 055). In der Versuchsanlage wurde als Katalysator Di-2-ethylhexylphosphat eingesetzt. In den unteren Teil des Rührreaktors wird durch Leitung 6 mit gasförmigem Stickstoff verdünnter Wasserdampf als zweiter Reaktand eingeführt. Die bei der Reaktion entstehenden Abgase, insbesondere CO₂, werden durch die Leitung 7 vom Kopf des Rührbehälters 1 abgeführt. Das bei der Reaktion entstehende Produkt, nämlich das Biuretgruppen aufweisende Polyisocyanat, wird dem unteren Ende des Behälters 1 durch Leitung 8 entnommen und über die Pumpe 9 einem Auffangbehälter 10 zugeführt. Im Rührbehälter 1 ist ein um eine senkrechte Achse 11 drehbarer Scheibenrührer 12 angeordnet. Die Temperatur im Rührbehälter 1 wird durch einen diesen umschließenden beheizbaren Mantel 13 eingestellt.

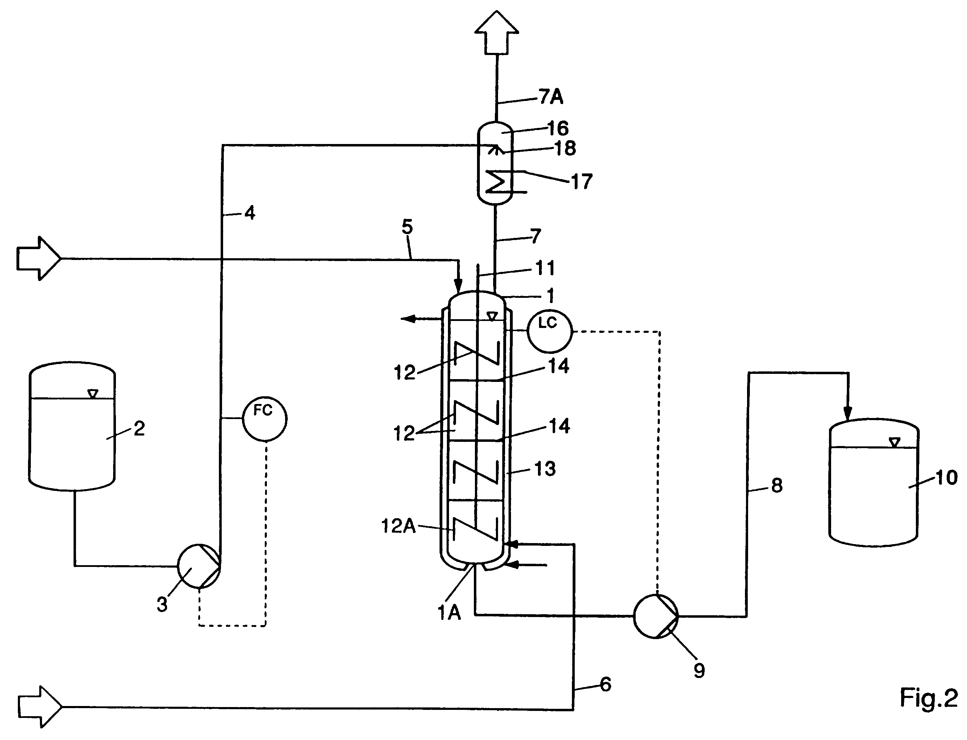

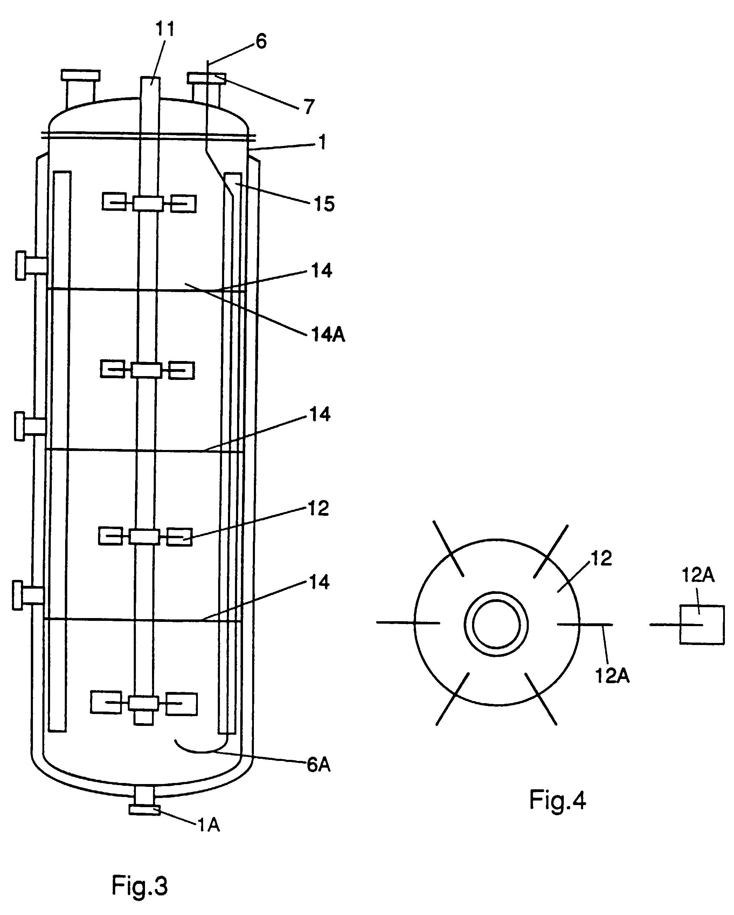

Bei der in Fig. 2 dargestellten erfindungsgemäßen Anlage ist der hier ebenfalls allgemein mit 1 bezeichnete Rührbehälter kaskadenförmig aufgebaut, wie dies im einzelnen der Fig. 3 zu entnehmen ist. In diesem Behälter sind an der parallel zur Längsachse des Behälters 1 verlaufenden Drehachse 11 vier Scheibenrührer 12 im Abstand voneinander angeordnet. Zwischen ihnen befinden sich drei Stromstörscheiben 14, die an der Wand der Rührbehälters 1 befestigt sind und eine kreisrunde Mittelöffnung aufweisen. In dem in Fig. 3 dargestellten Ausführungsbeispiel hat der Rührbehälter 1 einen Innendurchmesser von 100 mm. Die Öffnung der Stromstörscheiben 14 hat einen Durchmesser von 52 mm. Die Scheibenrührer 12, von denen einer in Fig. 4 dargestellt ist, können unterschiedliche Abmessungen haben. In der Regel werden Norm-Scheibenrührer verwendet. Im vorliegenden Falle hat die Scheibe des unteren Scheibenrührers 12 einen Durchmesser von 37,5 mm. Der Außendurchmesser einschließlich der senkrechten Rührflächen 12A beträgt 50 mm. Die Rührflächen 12A sind rechteckig ausgebildet mit einer Höhe von 10 mm und einer Breite von 12.5 mm. Die drei darüber angeordneten Scheibenrührer 12 haben einen Innendurchmesser von 30 und einen Außendurchmesser von 40 mm, wobei die Abmessung der Scheiben 8 x 10 mm beträgt. Im Inneren des Rührbehälters 1 sind vier Stromstörleisten 15 parallel zur Längsachse des Rührbehälters in einem Winkelabstand von je 90° bei einem Wandabstand von 1 mm angeordnet. Die Drehachse 11 mit den auf ihr angeordneten Scheibenrührern 12 dreht sich mit 500 bis 900 Umdrehungen pro Minute.

Auch bei der in Fig. 2 dargestellten erfingungsgemäßen Anlage wird dem Rührbehälter 1 aus dem Behälter mit Hilfe der Pumpe 3 durch die Leitung 4 der Reaktand HDI zugeführt. Die Leitung 4 führt hier über einen Injektor 18 zunächst in einen Kühlbehälter 16, der einen Kühler 17 aufweist. Mit diesem Kühler wird das aus dem Rührbehälter 1 durch Leitung 7A abströmende Abgas gekühlt. Eine Abscheidung von Rückständen wird hierdurch verhindert. Aus dem oberen Teil des Kühlbehälters 16 strömt das im wesentlichen aus CO₂ bestehende Abgas durch Leitung 7A ab. Das durch die Leitung 6 zuströmende Wasserdampf-/Stickstoffgemisch wird dem unteren Ende des Rührbehälters 1 zugeführt. Dies geschieht in der in Fig. 3 dargestellten Ausführungsform in der Weise, daß die Leitung 6 zwar am oberen Ende des Rührbehälters eintritt, das Ausströmen des Dampfes aber am unteren Ende des Rührbehälters 1 bei 6A erfolgt. Das Produkt wird dem unteren Ende des Rührbehälters 1 bei 1A entnommen und mit Hilfe der Pumpe 9 über die Leitung 8 dem Auffangbehälter 10 zugeleitet.

Sowohl die in Fig. 1 dargestellte bekannte Anlage wie auch die in Fig. 2 dargestellte erfindungsgemäße Anlage sind kontinuierlich und halbkontinuierlich betrieben worden. Bei der halbkontinuierlichen Fahrweise wird HDI und Katalysator vorgelegt und aufgeheizt. Anschließend wird kontinuierlich das Gemisch aus Wasserdampf und Stickstoff eingeleitet. Die Umsetzung dauert dabei insgesamt ca. 3 bis 4 Stunden.

Bei der Durchlauf-Fahrweise wird kontinuierlich HDI und Katalysator in den Reaktor 1 dosiert und parallel dazu das Gemisch aus Wasserdampf und Stickstoff eingeleitet. Das Rohprodukt aus HDI-Biuret-Oligomeren und überschüssigem Monomeren wird kontinuierlich in den Behälter 10 ausgetragen. Das Rohprodukt wird anschließend mittels Destillation aufgearbeitet.

Mit den zuvor beschriebenen Anlagen mit einfachem Rührbehälter (Fig. 1) und kaskadenförmigem Rührbehälter (Fig. 2) wurden kontinuierliche und diskontinuierliche Versuche durchgeführt. Es zeigte sich, daß der Umsatz des eingesetzten Wassers sowohl beim kontinuierlichen als auch beim diskontinuierlichen Verfahren im kaskadenförmigen Rührbehälter deutlich höher ist. Dies ist am Unterschied zwischen dem "realen NCO-Wert" und dem "idealen NCO-Wert" des Rohprodukts zu erkennen, die in den nachfolgenden Tabellen 1 und 2 aufgeführt sind. Der "ideale NCO-Wert" kann unter der Annahme berechnet werden, daß 1 Mol Wasser genau 3 Mol NCO-Gruppen umsetzt. Ist der reale Umsatz niedriger als der ideale, also der NCO-Wert höher (reines HDI besitzt 50% NCO), dann konnte nicht alles Wasser umgesetzt werden und der verwendete Reaktor ist in seiner Wirkungsweise nicht optimal. Ein niedrigerer NCO-Wert als der "ideale NCO-Wert" bedeutet zusätzliche Nebenreaktionen.

Die bei den Versuchen mit dem erfindungsgemäßen Kaskaden-Rührbehälter und einem einfachen Rührbehälter durchgeführten Versuche haben zu den in den nachfolgenden Tabellen angeführten Ergebnissen geführt.

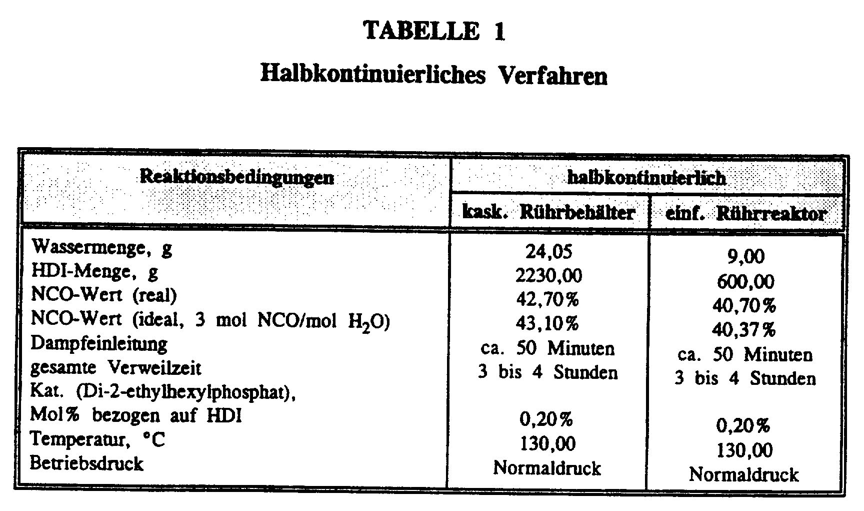

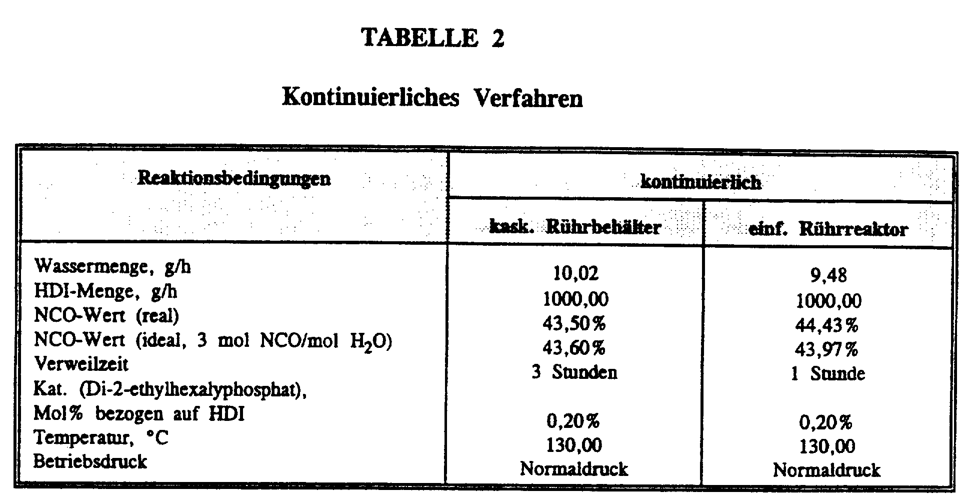

Insgesamt ist den Versuchsergebnisen zu entnehmen, daß das erfindungsgemäße Verfahren mit der hierfür geschaffenen neuen Vorrichtung zu deutlich besseren Ergebnissen führt, als die mit einfachem Rührkessel durchgeführten bekannten Verfahren.

## Patentansprüche

1. Verfahren zur Herstellung von (cyclo)aliphatischen Biuretgruppen enthaltenden Polyisocyanaten aus (cyclo)aliphatischen Diisocyanaten und Wasserdampf oder einer wasserabspaltenden Substanz als Reaktanden, die in einer Reaktoranordnung vermischt werden, dadurch gekennzeichnet, daß die Reaktanden im Gegenstrom durch eine aus mindestens zwei Stufen bestehende kaskadenförmige Reaktionsanordnung geführt werden.

2. Verfahren nach Anspruch 1, dadurch gekennzeichnet, daß die Verteilung der Gasphase im flüssigen Reaktanden durch in den Rührreaktor eingebaute Störelemente verstärkt wird.

3. Verfahren nach Anspruch 2, dadurch gekennzeichnet, daß als Störelemente mit Abstand voneinander angeordnete, mit zentralen Öffnungen versehene, Scheiben verwendet werden.

4. Verfahren nach Anspruch 2 oder 3, dadurch gekennzeichnet, daß als Störelemente in Längsrichtung des Rührreaktors verlaufende Störleisten verwendet werden.

5. Verfahren nach einem der vorherigen Ansprüche, dadurch gekennzeichnet, daß dem zugeführten Wasserdampf 10 bis 95 Vol.% Stickstoff und/oder Kohlendioxid beigemischt werden.

6. Verfahren nach einem der vorhergehenden Ansprüche, dadurch gekennzeichnet, daß eine Reaktionstemperatur von 60 bis 200 °C, vorzugsweise von 100 bis 150 °C, eingestellt wird.

7. Verfahren nach einem der vorhergehenden Ansprüche, dadurch gekennzeichnet, daß das am Kopfende des Rührreaktors abströmende Abgas mit kaltem (cyclo)aliphatischen Diisocyanat gespült wird, das anschließend dem Verfahren zugeführt wird.

8. Vorrichtung zur Durchführung des Verfahrens nach einem der vorhergehenden Ansprüche, dadurch gekennzeichnet, daß der Rührreaktor (1) einen senkrecht stehenden rohrförmigen Behälter aufweist, in dem parallel zur Längsachse eine Antriebsachse (11) drehbar befestigt ist, an der mindestens zwei Scheibenrührer (12) mit Abstand voneinander befestigt sind und daß zwischen diesen an der Innenwand des Behälters (1) befestigte Stromstörscheiben (14) angeordnet sind, die eine zentrale Öffnung (14A) aufweisen.

9. Vorrichtung nach Anspruch 8, dadurch gekennzeichnet, daß 2 bis 6 Scheibenrührer (12) und 1 bis 5 Stromstörscheiben (14) vorgesehen sind.

10. Vorrichtung nach Anspruch 8 oder 9, dadurch gekennzeichnet, daß an der Innenwand des Rührreaktors (1) parallel zu dessen Längsachse verlaufende radial sich nach innen erstreckende Stromstörleisten (15) angeordnet sind.

11. Vorrichtung nach Anspruch 10, dadurch gekennzeichnet, daß die Stromstörleisten (15) mit Abstand zur Innenwand des Reaktors (1) befestigt sind.

12. Vorrichtung nach Anspruch 10 oder 11, dadurch gekennzeichnet, daß mindestens vier im gleichen Winkelabstand voneinander angeordnete Störleisten (15) vorgesehen sind.

13. Vorrichtung nach einem der Ansprüche 8 bis 12, dadurch gekennzeichnet, daß der Rührreaktor (1) von einem beheizbaren Mantel (13) umschlossen ist.

14. Vorrichtung nach einem der Ansprüche 8 bis 13, dadurch gekennzeichnet, daß das Kopfende des Rührreaktors (1) mit einem Kühlbehälter (16) verbunden ist, in dessen unterem Teil eine Kühleinrichtung (17) angeordnet ist, oberhalb der ein Injektor (18) für den flüssigen Reaktanden zur Spülung der Kühlflächen angeordnet ist, über dem eine Abgasleitung (7A) mündet.

15. Vorrichtung nach einem der Ansprüche 8 bis 14 dadurch gekennzeichnet, daß das Verhältnis der Höhe des Rührreaktors zu dessen Durchmesser größer als 2, vorzugsweise größer als 4,5 ist.

## Claims

1. A process for preparing (cyclo)aliphatic polyisocyanates containing biuret groups from (cyclo)aliphatic diisocyanates and steam or a substance capable of splitting off water as reactants which are mixed in a reactor arrangement in which the reactants are conveyed in countercurrent through a cascade-type reaction arrangement comprising at least two stages.

2. A process as claimed in claim 1, wherein the dispersion of the gas phase in the liquid reactant is intensified by baffles installed in the stirred reactor.

3. A process as claimed in claim 2, wherein disks provided with central openings and arranged at a distance from one another are used as baffles.

4. A process as claimed in claim 2 or 3, wherein strip baffles running in the longitudinal direction of the stirred reactor are used as baffles.

5. A process as claimed in any of the preceding claims, wherein from 10 to 95 % by volume of nitrogen and/or carbon dioxide are mixed into the steam fed in.

6. A process as claimed in any of the preceding claims, wherein the reaction temperature is from 60 to 200°C, preferably from 100 to 150°C.

7. A process as claimed in any of the preceding claims, wherein the off-gas flowing from the top end of the stirred reactor is scrubbed with cold (cyclo)aliphatic diisocyanate which is subsequently fed to the process.

8. An apparatus for carrying out the process as claimed in any of the preceding claims, wherein the stirred reactor (1) which comprises an upright tubular vessel in which there is fixed, parallel to the longitudinal axis and able to rotate, a drive shaft (11) to which are fixed at least two disk stirrers (12) at a distance from one another, where between these disk stirrers (12) there are arranged disk baffles (14) fixed to the inner wall of the vessel (1) and having a central opening (14A).

9. An apparatus as claimed in claim 8, provided with from 2 to 6 disk stirrers (12) and from 1 to 5 disk baffles (14).

10. An apparatus as claimed in claim 8 or 9, wherein the inner wall of the stirred reactor (1) is provided with strip baffles (15) running parallel to the longitudinal axis of the reactor and extending radially inwards.

11. An apparatus as claimed in claim 10, wherein the strip baffles (15) are fixed so as to leave a gap between the baffle and the inner wall of the reactor (1) .

12. An apparatus as claimed in claim 10 or 11, wherein at least four strip baffles (15) equally spaced around the reactor wall are provided.

13. An apparatus as claimed in any of claims 8 to 12, wherein the stirred reactor (1) is surrounded by a heatable jacket (13).

14. An apparatus as claimed in any of claims 8 to 13, wherein the top end of the stirred reactor (1) is connected to a cooling vessel (16) in the lower part of which there is arranged a cooler (17) above which there is arranged an injector (18) for the liquid reactant for rinsing the cooling surfaces, and above the injector there opens an off-gas line (7A).

15. An apparatus as claimed in any of claims 8 to 14, wherein the ratio of the height of the stirred reactor to the diameter thereof is greater than 2, preferably greater than 4.5.

## Revendications

1. Procédé de préparation de polyisocyanates contenant des groupes biuret (cyclo)aliphatiques à partir de diisocyanates (cyclo)-aliphatiques et de vapeur d'eau ou d'une substance éliminant de l'eau, à titre de réactifs, qui sont mélangés dans un agencement de réacteur, caractérisé en ce que les réactifs sont guidés à contre-courant à travers un agencement de réaction en forme de cascade constitué d'au moins deux étages.

2. Procédé suivant la revendication 1, caractérisé en ce que la répartition de la phase gazeuse dans le réactif liquide est renforcée par des éléments perturbateurs incorporés dans le réacteur d'agitation.

3. Procédé suivant la revendication 2, caractérisé en ce que, comme éléments perturbateurs, on utilise des disques agencés à distance l'un de l'autre et pourvus d'ouvertures centrales.

4. Procédé suivant l'une des revendications 2 et 3, caractérisé en ce que, comme éléments perturbateurs, on utilise des barres perturbatrices qui s'étendent en direction longitudinale du réacteur d'agitation.

5. Procédé suivant l'une des revendications précédentes, caractérisé en ce qu'on mélange à la vapeur d'eau alimentée 10 à 95% en volume d'azote et/ou de dioxyde de carbone.

6. Procédé suivant l'une des revendications précédentes, caractérisé en ce qu'une température réactionnelle de 60 à 200°C, de préférence de 100 à 150°C, est ajustée.

7. Procédé suivant l'une des revendications précédentes, caractérisé en ce que le gaz résiduaire qui s'échappe à l'extrémité supérieure du réacteur d'agitation est rincé par du diisocyanate (cyclo)-aliphatique froid qui est ensuite amené au procédé.

8. Dispositif pour la mise en oeuvre du procédé suivant l'une des revendications précédentes, caractérisé en ce que le réacteur d'agitation (1) présente un réservoir tubulaire disposé verticalement dans lequel est fixé, parallèlement à l'axe longitudinal, un axe d'entraînement (11) de manière à pouvoir pivoter, axe sur lequel au moins deux agitateurs en forme de disques (12) sont fixés à distance l'un de l'autre et en ce qu'entre ceux-ci sont agencés des disques perturbateurs de courant (14) qui sont fixés sur la paroi interne du réservoir (1) et qui présentent une ouverture centrale (14A).

9. Dispositif suivant la revendication 8, caractérisé en ce que 2 à 6 agitateurs en forme de disques (12) et 1 à 5 disques perturbateurs de courant (14) sont prévus.

10. Dispositif suivant l'une des revendications 8 et 9, caractérisé en ce que, sur la paroi interne du réacteur d'agitation (1), sont agencées des barres perturbatrices de courant (15) qui s'étendent radialement vers l'intérieur et sont disposées parallèlement à l'axe longitudinal du réacteur.

11. Dispositif suivant la revendication 10, caractérisé en ce que les barres perturbatrices de courant (15) sont fixées à distance de la paroi interne du réacteur (1).

12. Dispositif suivant l'une des revendications 10 et 11, caractérisé en ce qu'au moins quatre barres perturbatrices (15), agencées à la même distance angulaire l'une de l'autre, sont prévues.

13. Dispositif suivant l'une des revendications 8 à 12, caractérisé en ce que le réacteur d'agitation (1) est entouré par une enveloppe chauffable (13).

14. Dispositif suivant l'une des revendications 8 à 13, caractérisé en ce que l'extrémité supérieure du réacteur d'agitation (1) est reliée à un récipient de refroidissement (16) dans la partie inférieure duquel est agencé un dispositif de refroidissement (17) au-dessus duquel est agencé un injecteur (18) des réactifs liquides pour le rinçage des surfaces de refroidissement, un conduit à gaz résiduaire (7A) débouchant au-dessus de l'injecteur.

15. Dispositif suivant l'une des revendications 8 à 14, caractérisé en ce que le rapport entre la hauteur du réacteur d'agitation et son diamètre est supérieur à 2, de préférence supérieur à 4,5.
